(19)

Europäisches
Patentamt
European
Patent Office
Office européen
des brevets

(11) **EP 3 513 730 A1**

(12) **EUROPEAN PATENT APPLICATION**

(43) Date of publication:
**24.07.2019 Bulletin 2019/30**

(51) Int Cl.:
**A61B 6/00** *(2006.01)* **G06T 7/00** *(2017.01)*
**G06T 7/10** *(2017.01)*

(21) Application number: **18152355.6**

(22) Date of filing: **18.01.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **BALTRUSCHAT, Ivo
5656 AE Eindhoven (NL)**

• **KNOPP, Tobias
5656 AE Eindhoven (NL)**
• **NICKISCH, Hannes
5656 AE Eindhoven (NL)**
• **SAALBACH, Axel
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **SYSTEM AND METHOD FOR IMAGE DECOMPOSITION OF A PROJECTION IMAGE**

(57)    The disclosure relates to a system for image decomposition of an anatomical projection image. The system comprises a data processing system which implements a decomposition algorithm of an projection image which is generated by irradiating a part of a subject with imaging radiation. A body portion within the irradiated part is a three-dimensional attenuation structure of an attenuation of the imaging radiation, wherein the attenuation structure represents a member of a predefined class of attenuation structures of the decomposition algorithm, thereby representing a classification of the body portion. The data processing system decomposes the projection image data using the classification of the attenuation structure. The decomposition of the projection image data substantially separates the contribution of the classified body portion to the projection image from the contribution of a further body portion of the subject to the projection image. The further body portion overlaps with the classified body portion in the projection image.

Fig. 3

**Description**

FIELD OF THE INVENTION

The present invention relates to a system and a method for analysis of projection images. More specifically, the present invention relates to a system and method for decomposition of projection images using predefined classes.

BACKGROUND OF THE INVENTION

Projection radiography is a widely adopted technique for medical diagnosis. It relies on projection images which are acquired from the patient. The projection images are generated using X-ray radiation which are emitted by an X-ray radiation source and which pass through a body portion of the patient. The X-ray radiation is attenuated by interaction with the different tissue types and bones of the body portion. A detector is arranged behind the body portion in relation to the X-ray radiation source. The detector absorbs the X-ray radiation remaining behind the patient and converts it into a projection image which is indicative of the X-ray attenuation caused by the patient.

A typical problem that arises when analyzing X-ray images is that the projection image of an anatomical or functional portion of the body, which is to be inspected, typically is obstructed due to other objects in an image, such as bones. This renders image analysis more difficult, often requiring profound expert knowledge and experience. By way of example, in the context of nodule detection using X-ray imaging, the radiologist conventionally has to consider that the appearance of a nodule in the image can be influenced by image contributions of the ribs, the spine, vasculature and other anatomical structures.

In view of this problem, the development of X-ray computer tomography has brought significant advances for X-ray based diagnosis. The computer tomography imaging system typically includes a motorized table which moves the patient through a rotating gantry on which a radiation source and a detector system are mounted. Data which is acquired from a single CT imaging procedure typically consist of either multiple contiguous scans or one helical scan. Using reconstruction algorithms volumetric (3D) representations of anatomical structures or cross-sectional images ("slices") through the internal organs and tissues can be obtained from the CD imaging data.

However it has been shown that CT scans can deliver 100 to 1,000 times higher dose compared to the dose delivered when acquiring a single X-ray projection image.

Accordingly, there is a need for a system and a method which allows for a more efficient diagnosis based on medical projection images.

SUMMARY OF THE INVENTION

Embodiments of the present disclosure provide a system for image decomposition of an anatomical projection image, the system comprising a data processing system which implements a decomposition algorithm. The decomposition algorithm is configured to read projection image data representing a projection image generated by irradiating a subject with imaging radiation. An irradiated body portion of the subject is a three-dimensional attenuation structure of an attenuation of the imaging radiation. The attenuation structure represents a member of a predefined class of attenuation structures of the decomposition algorithm, thereby representing a classification of the attenuation structure. The data processing system is further configured to decompose the projection image using the classification of the attenuation structure. The decomposition of the projection image decomposes between a contribution of the classified body portion to the projection image and a contribution of a further body portion of the subject to the projection image. The further body portion at least partially overlaps with the classified body portion in the projection image.

Thereby, based on a projection image, such as an X-ray projection image, a decomposition image of a body portion, such as the heart, can be obtained in which obstructing effects due to other body portions, such as the rib cage, are suppressed or even eliminated. Notably, in the field of X-ray analysis, this allows medical diagnosis based on low-dose projection radiology without the need to conduct complex and costly 3D X-ray reconstruction procedures. Such 3D X-ray reconstruction procedures require a complex CT-scanner, are time-consuming and cause a considerable amount of radiation exposure to the patient.

Accordingly, the proposed system allows decomposition of a 2D projection image into functionally meaningful constituents.

The data processing system may include a processor configured to perform the operations required to perform the separation algorithm. The data processing system may be a stand-alone data processing system, such as a stand-alone computer, or a distributed data processing system.

The projection image may be generated using projection imaging. In order to perform the projection imaging, a radiation source may be provided which is substantially a point source and which emits imaging radiation which traverses a part of the subject's body before being incident on a radiation detector which is configured to detect the imaging radiation. It is conceivable that more than one point sources are provided such as in scintigraphy. The intensity of each of the image points on the detector may depend on a line integral of local attenuation coefficients along a path of the incident ray. The line integral may represent an absorbance of the imaging radiation. Thereby, the projection image may be indicative of a two-dimensional absorbance distribution. The incident ray may travel substantially undeflected between the point source and the detector. The radiation source may be substantially a point source. It is conceivable that the radiation source is located within the subject's body, such as in scintigraphy.

The projection image may be generated using electromagnetic radiation (such as X-ray radiation and/or Gamma radiation). When X-ray radiography and/or scintigraphy is used for imaging, imaged body portions may attenuate the electromagnetic radiation used for generating the projection image. It is further conceivable that the projection image is generated using sound radiation as imaging radiation, in particular ultrasound radiation. A frequency of the ultrasound radiation may be within a range of between 0.02 and 1 GHz, in particular between 1 and 500 MHz. The imaging radiation may be generated using an acoustic transducer, such as a piezoelectric transducer.

The attenuation structure may be defined as a body portion, wherein within the body portion, the local absorbance is detectably different compared to adjacent body portions surrounding the attenuation structure. The attenuation structure may be defined by attenuation contrast. By way of example, at each point within the attenuation structure, the local attenuation exceeds the local attenuation of the adjacent body portions which surround the attenuation structure by a factor of more than 1.1 or by a factor of more than 1.2. Further by way of example, at each point within the attenuation structure, the local attenuation is less than the local attenuation of the adjacent body portions by a factor of less than 0.9 or by a factor of less than 0.8.

The data processing system may be configured to classify the body portion to obtain the classification. The data processing system may be configured to generate, using the projection image, one or more decomposition images. The decomposition images may represent a decomposition of the projection image into contributions of different body portions to the projection image. The different body portions may represent different classifications. Each of the decomposition images may show a contribution of a body portion, wherein a contribution of one or more other body portions is suppressed or eliminated.

According to an embodiment, the body portion is an anatomically and/or functionally defined portion of the body. An anatomically defined portion of the body may be a bone structure and/or a tissue structure of the body. A functionally defined portion of the body may be a portion of the body which performs an anatomical function.

According to a further embodiment, the decomposition of the projection image includes determining, for the projection image, a contribution image which is indicative of the contribution of the classified body portion to the projection image. The contribution image may represent a contribution of the body portion to the attenuation of the imaging intensity.

According to an embodiment, the decomposition of the projection image comprises generating a plurality of decomposition images, each of which being indicative of a two-dimensional absorbance distribution of the imaging radiation, which may be measured in an image plane of the projection image. For each point in the image plane, a sum of the absorbance distributions of the decomposition images may correspond to an absorbance distribution of the projection image within a predefined accuracy. The data processing system may be configured to check whether the sum corresponds to the absorbance distribution within the predefined accuracy.

According to a further embodiment, the decomposition algorithm includes a machine learning algorithm for performing the decomposition of the projection image using the classification of the body portion. The machine learning algorithm may be configured for supervised or unsupervised machine learning. In particular, the data processing system may be configured for user-interactive supervised machine learning.

According to a further embodiment, the decomposition algorithm includes a nearest neighbor classifier. The nearest neighbor classifier may be patch-based.

According to an embodiment, the data processing system is configured to train the machine learning algorithm using volumetric image data. The volumetric image data may be acquired using X-ray computer tomography.

According to an embodiment, the machine learning algorithm includes an artificial neural network (ANN). The ANN may include an input layer, an output layer and one or more intermediate layers. The ANN may include more than 5, more than 10, or more than 100 intermediate layers. The number of intermediate layers may be less than 500.

According to an embodiment, the data processing system is configured for semi-automatic or automatic segmentation of a portion of the volumetric image data. The segmented portion may represent the body portion which is to be classified. The data processing system may be configured to calculate, using the volumetric image data, a simulated projection image of the irradiated part of the subject and/or a simulated projection image of the segmented portion of the volumetric image data. The simulated projection images may be calculated using a ray casting algorithm. The semi-automatic segmentation may be user-interactive. The simulated projection images may be simulated based on a same position and/or orientation of the point source and the detector compared to the projection image.

According to a further embodiment, the data processing system is further configured to decompose the projection image depending on one or more further projection images. Each of the further projection images may be a projection image showing the classified body portion. The projection images may have mutually different projection axes.

Embodiments provide a method for image decomposition of an anatomical projection image using a data processing system. The data processing system implements a decomposition algorithm. The method comprises reading projection image data representing a projection image generated by irradiating a subject with imaging radiation. An irradiated body portion of the subject is a three-dimensional attenuation structure of an attenuation of the imaging radiation. The attenuation structure is a member of a predefined class of attenuation structures of the decomposition algorithm, thereby representing a classification of the attenuation structure. The method further comprises decomposing the projection image using the classification of the attenuation structure. The decomposition of the projection image decomposes between a contribution of the classified body portion to the projection image and a contribution of a further body portion of the subject to the projection image. The further body portion at least partially overlaps with the classified body portion in the projection image.

According to a further embodiment, the method comprises training the decomposition algorithm. The training of the decomposition algorithm may be performed using volumetric image data.

According to a further embodiment, the method comprises segmenting the body portion to be classified from the volumetric image data. The method may further comprise calculating or simulating a projection image of the segmented body portion.

Embodiments of the present disclosure provide a program element for image decomposition of an anatomical projection image, which program element, when being executed by a processor, is adapted to carry out reading projection image data representing a projection image generated by irradiating a subject with imaging radiation. An irradiated body portion of the subject represents a three-dimensional attenuation structure which is a member of a predefined class of attenuation structures of the decomposition algorithm, thereby representing a classification of the attenuation structure. The program element is further adapted to carry out decomposing the projection image using the classification of the attenuation structure. The decomposition of the projection image decomposes between a contribution of the classified body portion to the projection image and a contribution of a further body portion of the subject to the projection image. The further body potion at least partially overlaps with the classified body portion in the projection image.

Embodiments of the present disclosure provide a computer readable medium having stored the computer program element of the previously described program element.

These and other aspects of the invention will be apparent from and elucidated with reference to the embodiments described hereinafter.

BRIEF DESCRIPTION OF THE DRAWINGS

Fig. 1 is a schematic illustration of an exemplary scenario of acquiring a projection radiograph to be processed by a data analysis processing system according to a first exemplary embodiment;

Fig. 2 is an exemplary projection radiograph obtained in the scenario of Fig. 1;

Fig. 3 is a schematic illustration of a decomposition of the protection radiograph, shown in Fig. 2, using a decomposition algorithm according to an exemplary embodiment;

Fig. 4A schematically illustrates the layer structure of an artificial neural network (ANN) of the exemplary decomposition algorithm;

Fig. 4B schematically illustrates an exemplary process for training the exemplary decomposition algorithm;

Fig. 5A schematically illustrates an exemplary process of obtaining a simulated projection radiograph for training the exemplary decomposition algorithm;

Fig. 5B schematically illustrates an exemplary method for obtaining a simulated decomposed image for training the exemplary decomposition algorithm;

Fig. 6 is a schematic illustration of an exemplary scenario of acquiring multiple projection radiographs for a data analysis processing system according to a second exemplary embodiment; and

Fig. 7 is a flowchart illustrating an exemplary method for image decomposition of an anatomical projection image.

DETAILED DESCRIPTION OF EMBODIMENTS

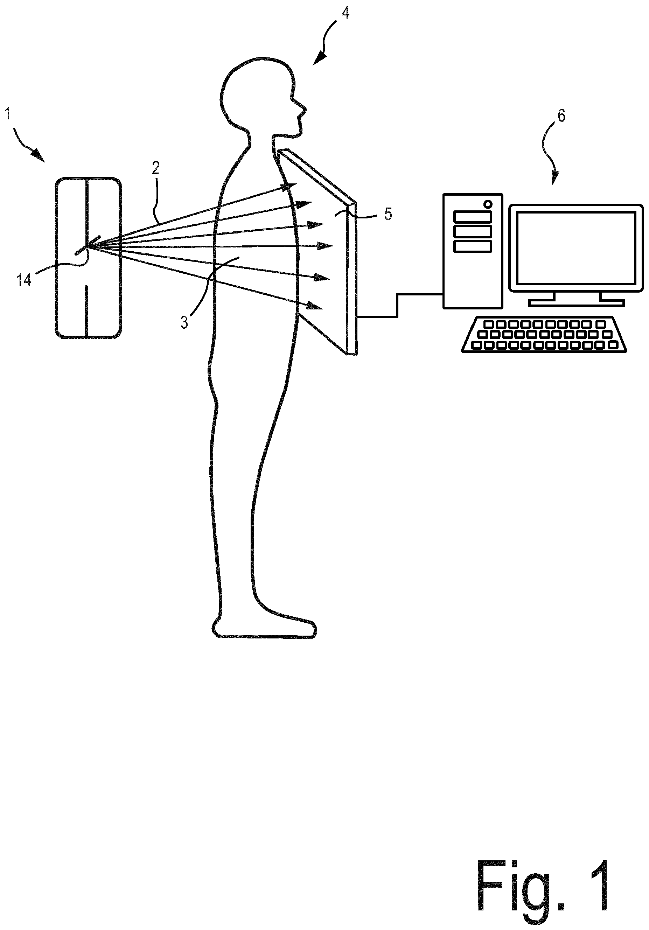

Fig. 1 is a schematic illustration of a projection X-ray chest radiography examination. An X-ray source 1, which is a tube assembly, is provided. The X-ray source 1 emits X-rays 2 toward a patient 4 to be examined so as to irradiate the patient's chest 3. The X-ray source 1 emits the X-rays from a small emission region 14 having a diameter of less than 10 millimeters, or less than 5 millimeters, which is considerably smaller than an extent of the imaged portion of the patient. Therefore, the X-ray source is a good approximation of a point radiation source. The chest 3 is arranged between the X-ray source 1 and an X-ray detector 5, which is configured to generate an image indicative of the intensity distribution of the X-rays incident on the X-ray detector 5. The X-ray detector may be an analog detector, such as a film, or a digital X-ray detector.

It is conceivable that the aspects and techniques of the present disclosure can be applied in conjunction with other imaging techniques which produce projection images, such as planar scintigraphy.

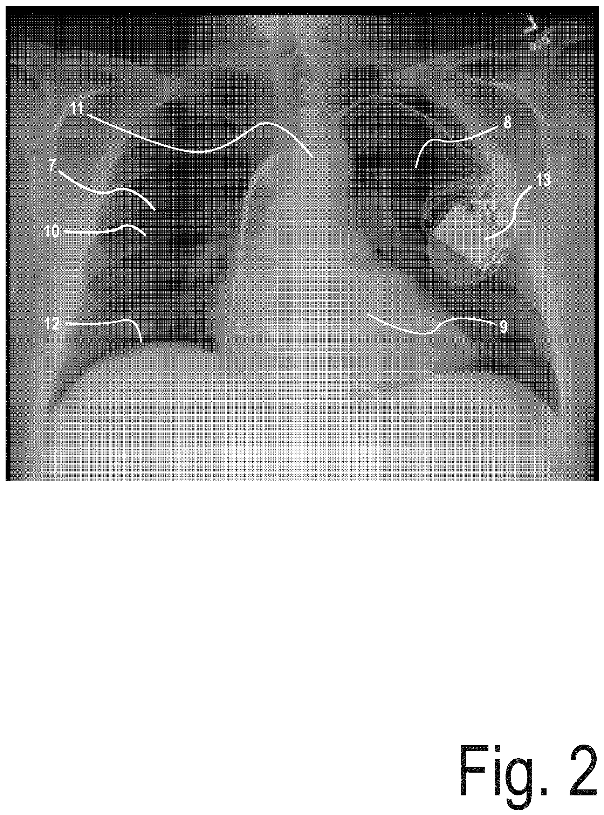

Fig. 2 shows an exemplary projection image, which has been acquired using the system illustrated in Fig. 1. As can be seen from Fig. 2, the protection image shows a plurality of two-dimensional structures, each of which representing a body portion which is an attenuation structure which attenuates the imaging X-ray radiation. In other words, in these body portions, the attenuation is significantly different from adjacent body portions, allowing them to be inspected using X-rays.

By way of example, the projection image of Fig. 2 shows two-dimensional structures representing the heart 9, the aortic arch 11, the right and left lobe of the lung 7, 8, the rib cage 10, the diaphragm 12 and a heart pacemaker 13. Some of the two-dimensional structures are overlapping in the projection image. Specifically, anatomical of functional portions of the body, which are to be inspected, such as the heart 9, are obstructed due to other objects in the projection image, such as the rib cage 10. This renders image analysis difficult, usually requiring profound expert knowledge and experience in order to obtain a reliable diagnosis.

However, it has been shown, that it is possible to decompose the projection image between the body portions. Thereby, for example, it is possible to obtain a contribution image showing the contribution of a single body portion to the projection image, wherein in the contribution image, the contributions of most or all of the remaining body portions are suppressed or even eliminated.

In order to perform the decomposition, a data processing system 6 (shown in Fig. 1) is provided which executes a decomposition algorithm. The decomposition algorithm is configured to decompose the projection image.

As will be explained in detail later, the decomposition algorithm uses one or a plurality of classes of three-dimensional attenuation structures. For the illustrated exemplary embodiment, examples for such classes include, but are not limited to, attenuation structures representing the heart, attenuation structures representing the rib cage and attenuation structures representing one or both lobes of the lung.

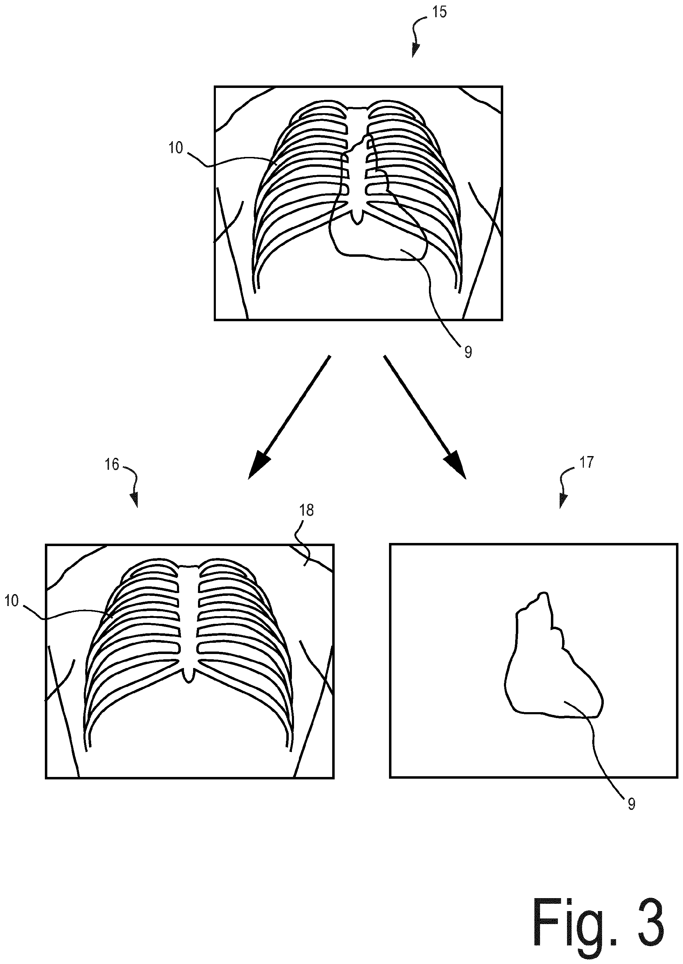

An example of a decomposition is described in the following with reference to Fig. 3. The decomposition algorithm provides at least two classes which include a first class for attenuation structures representing the heart 9 and a second class for attenuation structures representing the rib cage 10. The decomposition algorithm uses the projection image of the chest as input image 15 and generates, depending on the input image 15, a first decomposition image 16 and a second decomposition image 17. The first and the second decomposition images 16, 17 represent a decomposition between the two classes. Specifically, the first decomposition image 16 shows the contribution of the rib cage 10 to the input image 15 wherein contributions of the heart 9 are suppressed or eliminated. Further, the second decomposition image 17 shows the contribution of the heart 9 to the input image 15, wherein contributions of the rib cage 10 are suppressed or eliminated. One or both of the first and second decomposition images 16, 17 may show further contributions from further tissue portions 18 which may overlap with the two-dimensional structure representing the contribution of the heart 9 or the rib cage 10. Such tissue contributions may be acceptable, in particular if their contribution is weak compared to the contribution of the body portion of interest.

It is conceivable that the decomposition algorithm only provides one class, such as a class for attenuation structures of the heart, or more than two classes. Further, the decomposition algorithm may provide a class for remaining tissue portions of the irradiated part of the patient, which are not represented by other classes. Thereby, the classes may cover all body portions of the imaged part of the patient.

In the exemplary embodiment, the decomposition algorithm includes a machine learning algorithm for performing the decomposition of the protection image. The machine learning algorithm uses the classifications of the attenuation structures of one or more imaged body portions. In the exemplary embodiment, the machine learning algorithm is implemented using an artificial neural network (ANN). It is conceivable, however, that the decomposition is not a machine learning algorithm. The machine learning may be performed by supervised or unsupervised learning. Additionally or alternatively, it is conceivable that the decomposition algorithm includes a nearest neighbor classifier. The nearest neighbor classifier may be patch-based.

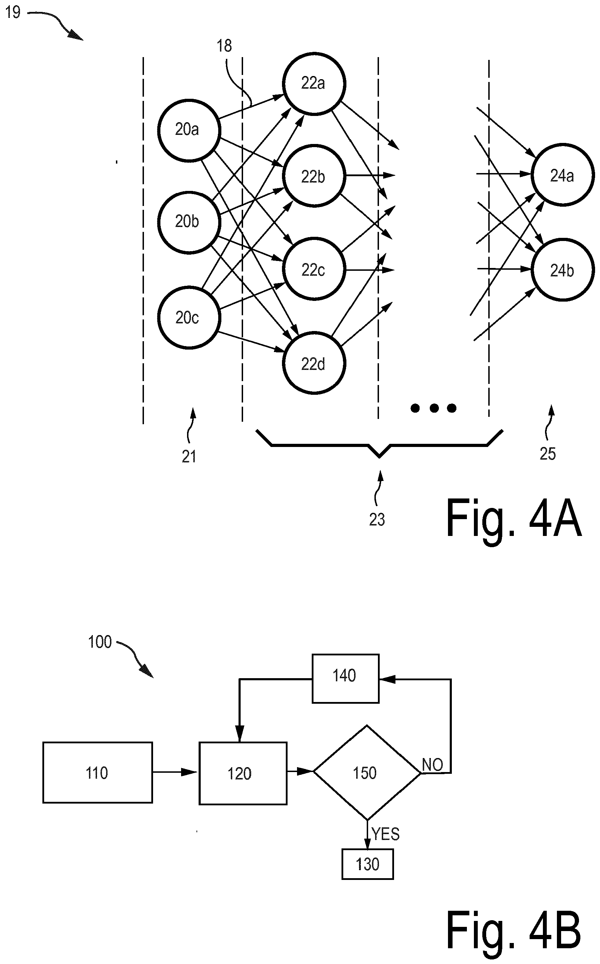

Fig. 4A is a schematic illustration of an ANN 19. The ANN 19 includes a plurality of neural processing units 20a, 20b, ... 24b. The neural processing units 20a, 20b, ... 24b are connected to form a network via a plurality of connections 18 each having a connection weight. Each of the connections 18 connects a neural processing unit of a first layer of the ANN 19 to a neural processing unit of a second layer of the ANN 19, which immediately succeeds or precedes the first layer. Thereby, the artificial neural network has a layer structure which includes an input layer 21, at least one intermediate layers 23 (also denoted as hidden layer) and an output layer 25. In Fig. 4a, only one of the intermediate layers 23 is schematically illustrated. The ANN 19 may include more than 5, or more than 10, or more than 100 intermediate layers.

It has been shown that using the ANN 19, it is possible to efficiently and reliably classify three-dimensional attenuation structures which are visible in the projection image.

Fig. 4B is an illustration of an exemplary training process 100. The training process 100 leads to a weight correction of the connection weights associated with the connections 18 (shown in Fig. 4A) of the ANN. As is illustrated in Fig. 4B, the training process 100 is iterative. In a first iteration of the training process 100, the connection weights are initialized to small random values. A sample image is provided 110 as an input to the ANN. The ANN decomposes the sample image to generate one or more decomposition images. By way of example, a first decomposition image may show the contribution of the rib cage, wherein contributions of other body portions, such as the heart, are suppressed or eliminated. A second contribution image shows the contribution of the heart wherein contributions of other body portions, such as the rib cage, are suppressed or eliminated. One or each of the decomposition images may show contributions from further tissue portions.

The ANN decomposes 120 the sample input image. Depending on a comparison between the decomposition images and reference decomposition images, it is determined whether the decomposition determined by the ANN has a required level of accuracy. If the decomposition has been achieved with a sufficient accuracy (150: YES), the training process 100 is ended 130. If the decomposition has not been achieved with sufficient accuracy (150: NO), the connection weights are adjusted 140. After adjustment of the connection weights, a further decomposition of the same or of different sample input images is performed as a next iteration.

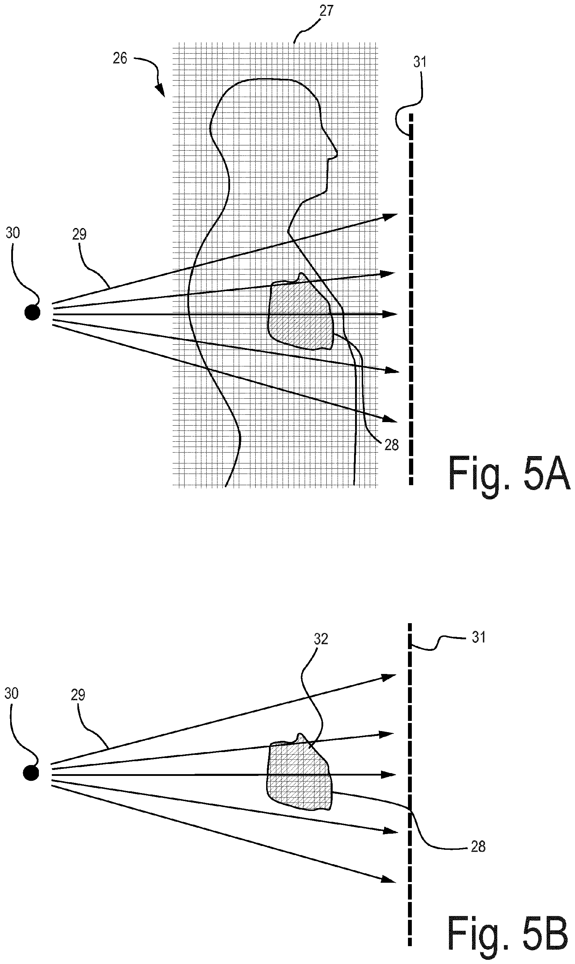

An exemplary process of generating sample input images and their corresponding decomposition images is described in the following with reference to Figs. 5A and 5B.

As is illustrated in Fig. 5A, the exemplary method uses a volumetric image data set 26 which includes a plurality of voxels 27. The volumetric image data set 26 may be, for example, generated by means of computer tomography using X-rays. In particular, the volumetric image data set 26 may represent a Digitally Reconstructed Radiograph (DRR).

Since in the exemplary embodiment, X-rays are used for generating the volumetric image data set 26, the volumetric image data show three-dimensional attenuation structures of an X-ray attenuation, such as the attenuation structure 28 (shown in Fig. 5A), which represents the heart of the patient. Each voxel 27 of the volumetric image data set 26 is a measure for the local attenuation coefficient at the respective voxel 27. Hence, a projection radiography image of the chest of the patient can be simulated by calculating, for each point $p_{x,y}$ on the detector, a line integral of local attenuation coefficients $\mu(l)$ (which are linear attenuation coefficients) along the path of the X-ray between the location $p_0$ of the point source and the point $p_{x,y}$ on the detector:

$$\mu_s(x,y) = \int_{p_0}^{p_{x,y}} \mu(l)\,dl = -\ln\left(\frac{I_{x,y}}{I_0}\right) \qquad \text{Equation 1,}$$

where x and $y$ are coordinates on the detector, $I_{x,y}$ is the intensity at the coordinates $y$ and $y$ and $I_0$ is the intensity which is incident on the patient's body. Equation 1 assumes that the effect of beam spreading is negligible. Equation 1 can be adapted to configurations where the effect of beam spreading is not negligible. Thereby, the values of the line integral $\mu_s(x,y)$ on the detector represent an absorbance distribution in the image plane of the projection image. As such, based on the volumetric image data, a simulated projection image can be obtained from the volumetric image data set 26 using a ray casting algorithm.

As is illustrated in Fig. 5B, a decomposition image showing the contribution of the heart can be simulated in a similar way by using the three-dimensional scattering structure 28 of the volumetric image data set 26 which corresponds to the heart without the surrounding voxels representing the remaining body portions. The voxels of the attenuation structure 28 are at a same position and orientation relative to the location of the point source 30 and the detector plane 31 as in Fig. 5A, i.e. when simulating the protection radiograph of the chest. Thereby, in a similar way as has been described with reference to Equation 1, an absorbance distribution in the image plane representing the heart can be obtained.

The voxels of the three-dimensional scattering structure 28 may be determined using a segmentation of the volumetric image data set 26. The segmentation may be automatic or semi-automatic. In particular, the data processing system (denoted with reference numeral 6 in Fig. 1) may be configured for user-interactive semi-automatic segmentation. The data processing system may be configured to receive user input and to perform the segmentation using the user input. The segmentation may be performed using a model-based segmentation algorithm and/or using an atlas-based segmentation algorithm.

Further, in a similar manner, simulated decomposition images of a plurality of further body portions, such as the rib cage and the lobes of the lung, can be obtained. In addition to these decomposition images, a further decomposition image which relates to all remaining portions of the body may be generated so that plurality of decomposition images are obtained, which cover each voxel in the volumetric data set 26 which has been traversed by X-rays.

Accordingly, for each point x, y on the detector screen, a pixel-wise sum of the absorbance distributions of the simulated decomposition images $\mu_{s,i}(x,y)$ (i = 1, ... n) yields the absorbance distribution of the simulated radiograph of the chest $\mu_s(x,y)$:

$$\mu_s(x, y) = \sum_{i=1}^{n} \mu_{s,i}(x, y) \quad \text{Equation 2.}$$

In the process 100 which is illustrated in Fig. 4D, for training the machine learning algorithm, the simulated projection radiograph of the chest, which has been calculated as has been described in connection with Fig. 5A, is used as an sample input image to the decomposition algorithm (step 110 in Fig. 4B). After the decomposition algorithm has calculated the decomposition images (step 120 in Fig. 4B) based on the sample input image, the decomposition images determined by the decomposition algorithm can be compared to the decomposition images simulated based on the volumetric image data set, as has been described in connection with Fig. 5B. This allows determination of whether or not the decomposition performed by the decomposition algorithm has the required accuracy (step 110 in Fig. 4B).

The decomposition of the sample input image (step 120 in Fig. 4B) includes generation of decomposition images of the body portions that have also been simulated based on the volumetric data set (i.e. in a manner as described in connection with Fig. 5B). In addition to the decomposition images of these body portions, the decomposition algorithm also generates a decomposition image, which relates to all the remaining portions of the body. Thereby, also for the decomposition images determined by the decomposition algorithm based on the sample input image, if the decomposition is ideally accurate, absorbance distributions $\mu_{d,i}$ *(x, y) (i = 1, ... n),* which correspond to the decomposition images obtained by the decomposition of the sample input image, sum up to the absorbance value of the simulated radiograph of the chest $\mu_s(x,y)$:

$$\mu_s(x, y) = \sum_{i=1}^{n} \mu_{d,i}(x, y) \quad \text{Equation 3.}$$

However, a deviation of the condition defined by Equation 3 by less than a preset level can still be regarded as acceptable in the assessment of accuracy in step 150 of Fig. 4B. In order to obtain a measure for the deviation, the data processing system may be configured to determine the L1-norm and/or the L2-norm between the absorbance distribution of the simulated radiograph of the chest (i.e. the left hand side of Equation 3) and the sum of the absorbance distributions of the decomposition images (i.e. the right hand side of Equation 3). As such, the L1-norm and/or the L2-norm may represent a cost function for training the machine learning algorithm, in particular the ANN.

Additionally or alternatively, the determination of whether the accuracy of the decomposition is acceptable (step 150 in Fig. 4B) may be performed on further criteria. In particular, the cost function for training the machine learning algorithm may depend on one or more of these further criteria. Such criteria may include the L1-norm and/or the L2-norm between the decomposition image (in particular the absorbance distribution of the decomposition image) of a body portion determined based on the volumetric image data set and the corresponding decomposition image (in particular the absorbance distribution of the decomposition image) of the body portion determined based on the sample input image. The L1 and/or the L2 norm of a plurality of body portions may be summed up.

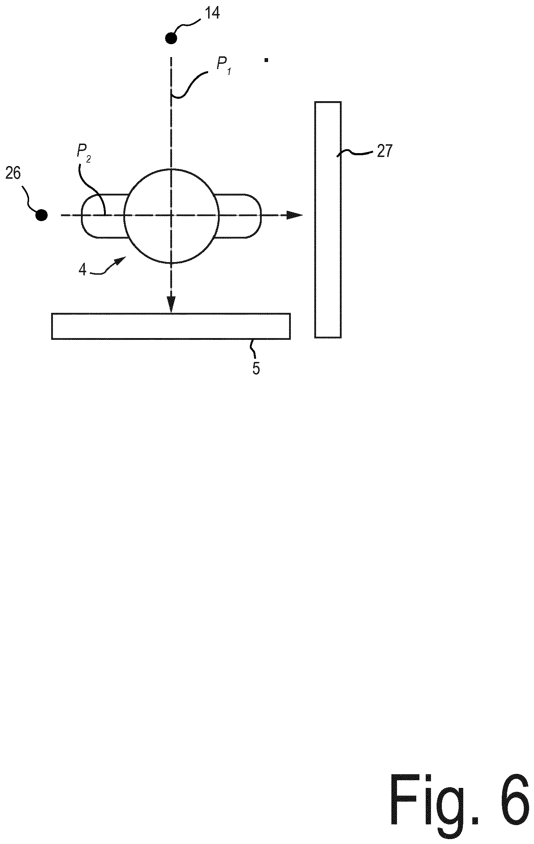

Fig. 6 illustrates a decomposition algorithm which is implemented in a data processing system according to a second exemplary embodiment. As in the decomposition algorithm of the first exemplary embodiment, also in the present embodiment, the decomposition algorithm is configured to decompose the projection image using the classification of an attenuation structure so that the obtained decomposition of the protection image substantially separates the body portion of the attenuation structure from further body portions of the subject which overlap with the body portion in the protection image.

The decomposition algorithm of the second exemplary embodiment is configured to perform the deposition depending on one or more further projection images. The first projection image and the one or more further projection images have mutually different imaging projection axes. The scenario for acquiring the projection images in the second exemplary embodiment is illustrated in Fig. 6, in which the longitudinal axis of the patient's body is oriented perpendicular to the paper plane. In the second exemplary embodiment, the first projection image is acquired with a first imaging projection axis $P_1$. A further projection image is acquired using a second imaging projection axis $P_2$ which is angled relative to the first imaging projection axis $P_1$. A projection axis may be defined to extend through the point source so as to be oriented perpendicular or substantially perpendicular to the active surface of the detector. In the imaging scenario which is illustrated in Fig. 6, the second projection image is acquired using a second radiation source which is configured as a point source so as to provide a second emission region 26 from which X-rays are emitted. Further, for acquiring the second projection image, a second detector 27 is provided. This allows simultaneous acquisition of both projection images, thereby alleviating using the information contained in the second projection image for decomposing the first projection image.

By way of example, the first imaging projection axis $P_1$ and the second imaging projection axis $P_2$ are angled relative to each other by about 10 degrees. In the first projection image, a portion of the heart is obstructed by ribs, whereas in the second projection image, this portion of the heart is not obstructed by ribs, allowing a finer analysis of the obstructed portion shown in the first projection image.

The decomposition of the projection image according to the second exemplary embodiment allows for a more reliable and a more precise decomposition of the first projection image. Furthermore, although multiple projection images are used by the data processing system, there is still a much lower radiation dose delivered to the patient, compared to conventional CT scans.

It is further to be noted that the orientation of the protection axes $P_1$ and $P_2$, as shown in Fig. 6, are only exemplary and are not intended to limit the application scope of the invention. The protection axes $P_1$ and $P_2$, as well as the number of projection images used may vary in other embodiments. By way of example an angle between the protection axes $P_1$ and $P_2$ may be greater than 5 degrees, greater than 10 degrees, greater than 15 degrees or greater than 20 degrees. The angle may be smaller than 180 degrees or smaller than 170 degrees.

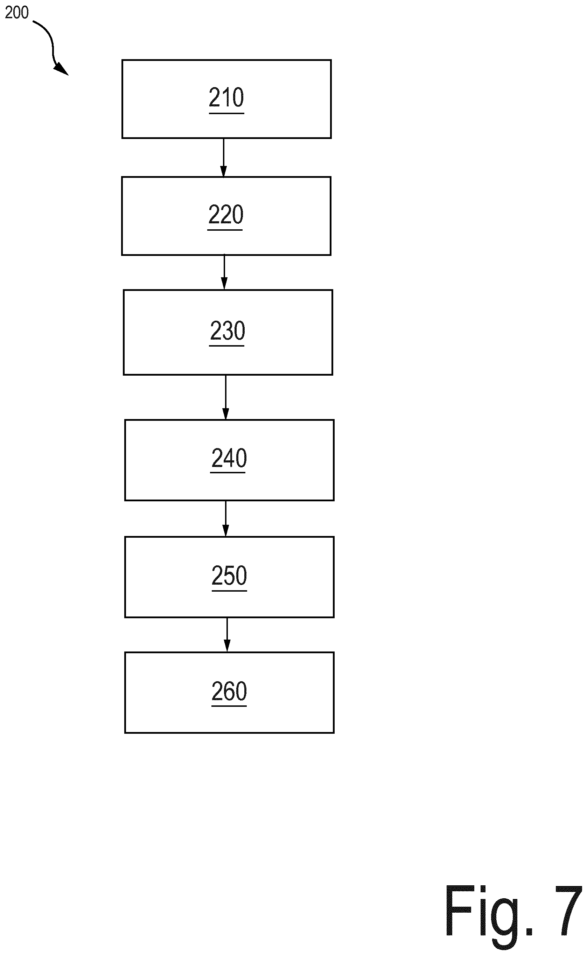

Fig. 7 is a flowchart which schematically illustrates an exemplary method for image decomposition of an anatomical protection image using a processing system, which executes a decomposition algorithm. Volumetric image data are acquired 210 in order to obtain data for training the decomposition algorithm, which is configured as a machine learning algorithm. The volumetric image data set may be acquired using X-ray computer tomography. In particular, the volumetric image data set may represent a digitally reconstructed radiograph (DRR). The volumetric image data set shows a body portion, which is later analyzed using X-ray protection images. The method further includes segmenting 220 the volumetric image data. The segmentation may be performed using the data processing system. The segmentation may performed using automatic or semi-automatic segmentation. The semi-automatic segmentation may be performed depending on user input received via an interface of the data processing system. The interface may include a graphical user interface of the data processing system. Depending on the segmented volumetric image data, a simulated projection image of an irradiated part of a patient and one or more simulated decomposed images of one or more body portions within the irradiated part are calculated 230. The simulated images may be calculated using a ray casting algorithm. Depending on the simulated images, the decomposition algorithm is trained 240. After the protection image has been acquired in medical examination procedures, the protection image data are read 250 by the data processing system. The data processing system then decomposes 260 the projection image data using classifications of the attenuation structures, which correspond to the body portions within the irradiated part of the patient. The classifications of the attenuation structures include assigning the attenuation structure to a predefined class of the decomposition algorithm. For each of the body portions, a decomposition image is generated. The decomposition image corresponds to a separated image, in which the respective body portion is separated from one or more of the further body portions. By way of example, the body portion of a decomposition image corresponds to the heart and the decomposition image shows the contribution of the heart, wherein a contribution of the rib cage is suppressed or even eliminated.

It has been shown that thereby, a system and a method is provided which allows for a more efficient diagnosis based on medical projection images.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In particular, although the invention has been described based on a projection radiograph, it can be applied to any imaging technique which results in a projection image. In the claims, the word "comprising" does not exclude other elements or steps, and the indefinite article "a" or "an" does not exclude a plurality. Any reference signs in the claims should not be construed as limiting the scope.

**Claims**

1. A system for image decomposition of an anatomical projection image, the system comprising a data processing system (6) which implements a decomposition algorithm configured to:

   read projection image data representing a projection image generated by irradiating a part of a subject with imaging radiation;
   wherein a body portion within the irradiated part is a three-dimensional attenuation structure of an attenuation of the imaging radiation, wherein the attenuation structure represents a member of a predefined class of attenuation structures of the decomposition algorithm, thereby representing a classification of the attenuation structure;
   wherein the data processing system (6) is further configured to
   decompose the projection image using the classification of the attenuation structure; and
   wherein the decomposition of the projection image decomposes between a contribution of the classified body portion to the projection image and a contribution of a further body portion in the irradiated part to the projection image, wherein the further body portion at least partially overlaps with the classified body portion in the projection image.

2. The system of claim 1, wherein the attenuation structure is an anatomically and/or functionally defined portion of the body.

3. The system of claim 1 or 2, wherein the decomposition of the projection image includes determining, for the projection image, a contribution image which is indicative of the contribution of the classified body portion to the projection image.

4. The system of any one of the preceding claims, wherein the decomposition of the projection image comprises generating a plurality of decomposition images (16, 17), each of which being indicative of a two-dimensional absorbance distribution of the imaging radiation;
wherein for each point in the image plane, a sum of the absorbance distributions of the decomposition images corresponds to an absorbance distribution of the projection image within a predefined accuracy.

5. The system of any one of the preceding claims, wherein the decomposition algorithm includes a machine learning algorithm for performing the decomposition of the projection image using the classification of the body portion.

6. The system of claim 5, wherein the machine learning algorithm includes an artificial neural network (ANN).

7. The system of claim 5 or 6, wherein the data processing system is configured to train the machine learning algorithm using volumetric image data.

8. The system of claim 7, wherein the data processing system is configured for semi-automatic or automatic segmentation of a portion of the volumetric image data representing the body portion, which is to be classified, from the volumetric image data and to calculate a simulated projection image of the segmented portion of the volumetric image data.

9. The system of any one of the preceding claims, wherein the data processing system is further configured to decompose the projection image depending on one or more further projection images, each of which being a projection image showing the classified body portion;
wherein the projection images have mutually different projection axes.

10. A method for image decomposition of an anatomical projection image using a data processing system (6) which implements a decomposition algorithm, the method comprising:

reading (250) projection image data representing a projection image generated by irradiating a part of a subject with imaging radiation;
wherein a body portion within the irradiated part is a three-dimensional attenuation structure of an attenuation of the imaging radiation, wherein the attenuation structure represents a member of a predefined class of attenuation structures of the decomposition algorithm, thereby representing a classification of the attenuation structure;
decomposing (260) the projection image using the classification of the attenuation structure;
wherein the decomposition of the projection image decomposes between a contribution of the classified body portion to the projection image and a contribution of a further body portion in the irradiated part to the projection image, wherein the further body portion at least partially overlaps with the classified body portion in the projection image.

11. The method of claim 10, further comprising training (240) the decomposition algorithm.

12. The method of claim 11, wherein the training (240) of the decomposition algorithm is performed using volumetric image data.

13. The method of claim 12, further comprising segmenting (220) the body portion to be classified from the volumetric image data and calculating (230) a projection image of the segmented body portion.

14. A program element for image decomposition of an anatomical projection image, which program element, when being executed by a processor, is adapted to carry out:

reading (250) projection image data representing a projection image generated by irradiating a part of a subject with imaging radiation;

wherein a body portion within the irradiated part is a three-dimensional attenuation structure of an attenuation of the imaging radiation, wherein the attenuation structure represents a member of a predefined class of attenuation structures of the decomposition algorithm, thereby representing a classification of the attenuation structure;

decomposing (260) the projection image using the classification of the attenuation structure;

wherein the decomposition of the projection image decomposes between a contribution of the classified body portion to the projection image and a contribution of a further body portion in the irradiated part to the projection image, wherein the further body portion at least partially overlaps with the classified body portion in the projection image.

**15.** A computer readable medium having stored the computer program element of claim 14.

Fig. 1

Fig. 2

Fig. 3

Fig. 4A

Fig. 4B

Fig. 5A

Fig. 5B

Fig. 6

200

```
┌─────────────────┐
│       210       │
└─────────────────┘
         │
         ▼
┌─────────────────┐
│       220       │
└─────────────────┘
         │
         ▼
┌─────────────────┐
│       230       │
└─────────────────┘
         │
         ▼
┌─────────────────┐
│       240       │
└─────────────────┘
         │
         ▼
┌─────────────────┐
│       250       │
└─────────────────┘
         │
         ▼
┌─────────────────┐
│       260       │
└─────────────────┘
```

# Fig. 7

Europäisches
Patentamt

European
Patent Office

Office européen
des brevets

**EUROPEAN SEARCH REPORT**

Application Number

EP 18 15 2355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/178378 A1 (YOUNG STEWART [DE] ET AL) 22 June 2017 (2017-06-22) * paragraph [0033] - paragraph [0068]; figures 1-6 * | 1-6,10, 14,15 | INV. A61B6/00 G06T7/00 G06T7/10 |
| X | US 2015/294182 A1 (KENIG TAL [IL] ET AL) 15 October 2015 (2015-10-15) * paragraph [0069] - paragraph [0141]; figures 1-11 * | 1-6,10, 14,15 | |
| X | US 2014/140603 A1 (HUO ZHIMIN [US] ET AL) 22 May 2014 (2014-05-22) * paragraph [0039] - paragraph [0052] * | 1-6,10, 14,15 | |
| X | US 2013/108135 A1 (HUO ZHIMIN [US] ET AL) 2 May 2013 (2013-05-02) * paragraph [0028] - paragraph [0045] * | 1-6,10, 14,15 | |

TECHNICAL FIELDS
SEARCHED      (IPC)

A61B
G06T

~~The present search report has been drawn up for all claims~~

| Place of search | Date of completion of the search | Examiner |
|---|---|---|
| Munich | 19 June 2018 | Martinez Möller, A |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

                                      
& : member of the same patent family, corresponding
     document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 10, 14, 15

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

# LACK OF UNITY OF INVENTION
## SHEET B

Application Number

EP 18 15 2355

The Search Division considers that the present European patent application does not comply with the requirements of unity of invention and relates to several inventions or groups of inventions, namely:

1. claims: 1-6, 10, 14, 15

   Details of the attenuation structure and the generated decomposition images, thus allowing to provide an adequate output to the user.
   ---

2. claims: 7, 8, 11-13

   Details of the training of the decomposition algorithm, thus allowing to provide an improved decomposition.
   ---

3. claim: 9

   Details of the usage of further projection images, thus allowing an accurate decomposition of obstructed portions in the projection image.
   ---

## ANNEX TO THE EUROPEAN SEARCH REPORT
## ON EUROPEAN PATENT APPLICATION NO.

EP 18 15 2355

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2018

| Patent document cited in search report | | | Publication date | Patent family member(s) | | | Publication date |
|---|---|---|---|---|---|---|---|
| US 2017178378 | A1 | | 22-06-2017 | CN | 106575436 | A | 19-04-2017 |
| | | | | EP | 3161803 | A1 | 03-05-2017 |
| | | | | JP | 2017518828 | A | 13-07-2017 |
| | | | | US | 2017178378 | A1 | 22-06-2017 |
| | | | | WO | 2015197738 | A1 | 30-12-2015 |
| US 2015294182 | A1 | | 15-10-2015 | EP | 2942751 | A1 | 11-11-2015 |
| | | | | US | 2015294182 | A1 | 15-10-2015 |
| US 2014140603 | A1 | | 22-05-2014 | NONE | | | |
| US 2013108135 | A1 | | 02-05-2013 | NONE | | | |

EPO FORM P0459

For more details about this annex : see Official Journal of the European Patent Office, No. 12/82